# FASCICULE DE BREVET EUROPEEN

(11) **EP 2 283 103 B1**
(45) Date de publication et mention de la délivrance du brevet: **26.11.2014**
(21) Numéro de dépôt: 09772678.0
(22) Date de dépôt: 14.05.2009
(51) Int. Cl.: C09K 3/30, C09K 5/04, C08J 9/14

(54) **COMPOSITIONS A BASE D'HYDROFLUOROOLEFINES**
FLUORKOHLENWASSERSTOFFE BASIERTE ZUSAMMENSETZUNGEN
COMPOSITIONS BASED ON HYDROFLUOROCARBONS

(30) Priorité: 11.06.2008 FR 0853861
(43) Date de publication de la demande: 16.02.2011
(73) Titulaire: Arkema France, 92700 Colombes (FR)
(72) Inventeur: RACHED, Wissam, F-69630 Chaponost (FR)
(74) Mandataire: Dang, Doris
(86) Numéro de dépôt international: PCT/FR2009/050891
(87) Numéro de publication internationale: WO 2010/000995

(56) Documents cités:
- WO-A-2006/094303
- WO-A-2008/009922
- US-A1- 2007 007 488

## Description

La présente invention concerne des compositions renfermant des hydrofluorooléfines et leurs utilisations comme fluides de transfert de chaleur, agents d'expansion, solvants et aérosols.

Les problèmes posés par les substances appauvrissant la couche d'ozone atmosphérique (ODP : ozone depletion potential) ont été traités à Montréal où a été signé le protocole imposant une réduction de la production et de l'utilisation des chlorofluorocarbures (CFC). Ce protocole a fait l'objet d'amendements qui ont imposé l'abandon des CFC et étendu la réglementation à d'autres produits, dont les hydrochlorofluorocarbones (HCFC).

L'industrie de la réfrigération et de la production d'air conditionné a beaucoup investi dans la substitution de ces fluides frigorigènes et c'est ainsi que les hydrofluorocarbures (HFC) ont été commercialisés.

Les (hydro)chlorofluorocarbures utilisés comme agents d'expansion ou solvants ont également été substitués par des HFC.

Dans l'industrie automobile, les systèmes de climatisation des véhicules commercialisés dans de nombreux pays sont passés d'un fluide frigorigène au chlorofluorocarbure (CFC-12) à celui de l'hydrofluorocarbure (1,1,1,2 tetrafluoroéthane : HFC-134a), moins nocif pour la couche d'ozone. Cependant, au regard des objectifs fixés par le protocole de Kyoto, le HFC-134a (GWP = 1300) est considéré comme ayant un pouvoir de réchauffement élevé. La contribution à l'effet de serre d'un fluide est quantifiée par un critère, le GWP (Global Warming Potentials) qui résume le pouvoir de réchauffement en prenant une valeur de référence de 1 pour le dioxyde de carbone.

Le dioxyde de carbone étant non-toxique, ininflammable et ayant un très faible GWP, a été proposé comme fluide frigorigène des systèmes de climatisation en remplacement du HFC-134a. Toutefois, l'emploi du dioxyde de carbone présente plusieurs inconvénients, notamment liés à la pression très élevée de sa mise en oeuvre en tant que fluide frigorigène dans les appareils et technologies existants.

Le document JP 4110388 décrit l'utilisation des hydrofluoropropènes de formule C₃HₘFₙ, avec m, n représentant un nombre entier compris entre 1 et 5 inclus et m + n = 6, comme fluides de transfert de chaleur, en particulier le tetrafluoropropène et le trifluoropropène.

Le document WO2004/037913 divulgue l'utilisation des compositions comprenant au moins un fluoroalcène ayant trois ou quatre atomes de carbone, notamment le pentafluoropropène et le tetrafluoropropène, de préférence ayant un GWP au plus de 150, comme fluides de transfert de chaleur.

Le document WO 2005/105947 enseigne l'ajout au tetrafluoropropène, de préférence le 1,3,3,3 tetrafluoropropène, d'un co-agent d'expansion tels que le difluoromethane (HFC-32), le pentafluoroéthane (HFC-125), le tetrafluoroéthane, le difluoroéthane, l'heptafluoropropane, l'hexafluoropropane, le pentafluoropropane, le pentafluorobutane, l'eau et le dioxyde de carbone.

Le document WO 2006/094303 divulgue une composition azéotropique contenant 70,4 % en poids du 2,3,3,3 tetrafluoropropène (1234yf) et 29,6 % en poids du 1,1,1,2 tetrafluoroéthane (HFC-134a). Ce document divulgue également une composition azéotropique contenant 91 % en poids du 2,3,3,3 tetrafluoropropène et 9 % en poids du difluoroéthane (HFC-152a).

Le document WO 2008/009922 divulgue des compositions de transfert de chaleur comprenant du pentafluoropropène, du tétrafluoropropène et au moins un réfrigérant choisi parmi le dioxyde de carbone, le difluorométhane, le 1,1,1,2-tétrafluoroéthane, le 3,3,3,-trifluoropropène, le 1,1,-difluoroéthane, le propane, le propène, l'isobutane ou le n-butane.

Le document US 2007/0007488 divulgue des compositions de transfert de chaleur comprenant au moins un fluoroalcène ayant un nombre de carbone compris entre 3 et 6.

La demanderesse a maintenant mis au point des compositions renfermant des hydrofluoropropènes ne présentant pas les inconvénients précités et ayant à la fois un ODP nul et un GWP inférieur à celui des HFC existants comme le R407C (mélange ternaire du HFC-134a (52 % en poids), HFC-125 (25 % en poids) et HFC-32 (23 % en poids)).

En outre, ces compositions sont quasi-azéotropiques.

Les compositions selon la présente invention sont caractérisées en ce qu'elles comprennent de 10 à 90 % en poids du 2,3,3,3 tetrafluoropropène, de 5 à 85 % en poids du HFC-134a et de 2 à 20 % en poids du HFC-152a.

Selon un mode préféré de l'invention, les compositions comprennent de 2 à 15 % en poids du HFC-152a, de 15 à 70 % en poids du 2,3,3,3 tetrafluoropropène et de 15 à 70 % en poids du HFC-134a.

Les compositions comprenant 10-11 % en poids du HFC-152a, 82-83 % en poids du 2,3,3,3 tetrafluoropropène et 6-7 % en poids du HFC-134a sont particulièrement intéressantes. Ces compositions sont azéotropiques et ont un point d'ébullition de -29,5°C (+ ou - 0,5°C) à la pression de 1 bar absolu.

Avantageusement, les compositions selon la présente invention contiennent essentiellement du 2,3,3,3 tetrafluoropropène, du HFC-134a et du HFC-152a comme hyfrofluorocarbures (saturés et insaturés).

Les compositions selon la présente invention peuvent être utilisées comme fluides de transfert de chaleur et conviennent particulièrement pour les systèmes à compression pour l'air conditionné et le chauffage notamment les pompes à chaleur, de préférence en remplacement du R407C et du HFC-134a. Ces compositions peuvent remplacer le R407C dans les nouvelles installations alors que pour le HFC-134a, le remplacement convient aussi bien pour les anciennes que pour les nouvelles installations.

Les compositions selon la présente invention peuvent comprendre un stabilisant du 2,3,3,3 tetrafluoropropène. Le stabilisant représente au plus 5 % en poids par rapport à la composition totale.

Comme stabilisants, on peut citer notamment le nitromethane, l'acide ascorbique, l'acide terephtalique, les azoles tels que le tolutriazole ou le benzotriazole, les composés phénoliques tels que le tocopherol, l'hydroquinone, le t-butyl hydroquinone, le 2,6-di-ter-butyl-4-methylphenol, les epoxydes (alkyl éventuellement fluoré ou perfluoré ou alkenyl ou aromatique) tels que les n-butyl glycidyl ether, hexanediol diglycidyl ether, allyl glycidyl ether, butylphenylglycidyl ether, les phosphites, les phosphates, les phosphonates, les thiols et lactones.

Les compositions selon la présente invention peuvent comprendre des lubrifiants tels que l'huile minérale, alkylbenzène, le polyalkylène glycol et polyvinyl éther.

Les compositions selon la présente invention sont en outre utilisables comme agents d'expansion, aérosols et solvants.

### PARTIE EXPERIMENTALE

Les performances des compositions selon l'invention dans les conditions de fonctionnement pour pompe à chaleur et climatisation sont données dans le tableau ci-dessous. Les valeurs des différents constituants (1234yf, 134a et 152a) sont données en pourcentage en poids.

| | |
|---|---|
| Température d'évaporation | : -5°C |
| Température de condensation | : 70°C |
| Température entrée compresseur | : 5°C |
| Température du liquide sous refroidi | : 65°C |
| Rendement isentropique du compresseur | : 70 % |

| | |
|---|---|
| Evap P : pression à l'évaporateur Cond P : pression au condenseur Taux : le taux de compression T sortie comp : température à la sortie compresseur COP : coefficient de performance et est défini, lorsqu'il s'agit d'une pompe à chaleur, comme étant le rapport de la puissance chaude utile fournie par le système sur la puissance apportée ou consommée par le système. | |

Les valeurs du COP sont supérieures aux valeurs obtenues avec le R407C et en outre, les compositions selon l'invention sont azéotropiques ou quasi-azéotropiques.

| **Produits** | | | **evap P (kpa)** | **cond P (kPa)** | **Taux (p/p)** | **T sortie comp** | **Capacité (KJ/m3)** | **COP** |
|---|---|---|---|---|---|---|---|---|
| **R407C** | | | 385,68 | 3442,55 | 8,93 | 126,60 | 1461 | 2,1 |
| | | | | | | | | |

| **1234yf** | **134a** | **152a** | | | | | | |
|---|---|---|---|---|---|---|---|---|
| | | | | | | | | |
| 90 | 5 | 5 | 266,96 | 1987,74 | 7,45 | 89,97 | 965,61 | 2,3 |
| 70 | 25 | 5 | 270,15 | 1996,67 | 7,39 | 90,96 | 986,45 | 2,3 |
| 50 | 45 | 5 | 266,26 | 2017,93 | 7,58 | 93,53 | 1010,98 | 2,3 |
| 30 | 65 | 5 | 257,14 | 2047,39 | 7,96 | 97,49 | 1044,55 | 2,3 |
| 10 | 85 | 5 | 245,44 | 2081,77 | 8,48 | 102,68 | 1092,91 | 2,4 |
| 80 | 10 | 10 | 268,52 | 1958,26 | 7,29 | 90,62 | 984,02 | 2,3 |
| 70 | 20 | 10 | 268,44 | 1958,04 | 7,29 | 91,26 | 992,13 | 2,3 |
| 60 | 30 | 10 | 266,65 | 1963,44 | 7,36 | 92,37 | 1001,44 | 2,3 |
| 50 | 40 | 10 | 263,40 | 1973,79 | 7,49 | 93,91 | 1012,82 | 2,3 |
| 40 | 50 | 10 | 258,99 | 1988,43 | 7,68 | 95,84 | 1027,18 | 2,3 |
| 30 | 60 | 10 | 253,77 | 2006,73 | 7,91 | 98,12 | 1045,34 | 2,4 |
| 20 | 70 | 10 | 248,05 | 2028,11 | 8,18 | 100,67 | 1067,93 | 2,4 |
| 10 | 80 | 10 | 242,07 | 2052,08 | 8,48 | 103,64 | 1095,38 | 2,4 |
| 80 | 5 | 15 | 267,94 | 1939,06 | 7,24 | 91,31 | 996,91 | 2,3 |
| 60 | 25 | 15 | 264,78 | 1932,24 | 7,30 | 92,94 | 1009,30 | 2,3 |
| 40 | 45 | 15 | 256,37 | 1953,90 | 7,62 | 96,48 | 1031,77 | 2,4 |
| 20 | 65 | 15 | 245,22 | 1998,02 | 8,15 | 101,67 | 1071,57 | 2,4 |
| 70 | 10 | 20 | 266,23 | 1915,88 | 7,20 | 92,71 | 1016,26 | 2,3 |
| 60 | 20 | 20 | 263,31 | 1910,88 | 7,26 | 93,72 | 1020,63 | 2,3 |
| 50 | 30 | 20 | 259,20 | 1914,87 | 7,39 | 95,26 | 1027,88 | 2,4 |
| 40 | 40 | 20 | 254,23 | 1927,16 | 7,58 | 97,26 | 1039,17 | 2,4 |
| 30 | 50 | 20 | 248,70 | 1946,87 | 7,83 | 99,60 | 1055,32 | 2,4 |
| 20 | 60 | 20 | 242,88 | 1973,05 | 8,12 | 102,61 | 1076,89 | 2,4 |

Le glissement de température à l'évaporateur est de 4°C pour le R407C tandis qu'il est au plus de 0,2°C pour les compositions selon l'invention.

## Revendications

1. Compositions comprenant de 10 à 90 % en poids du 2,3,3,3 tetrafluoropropène, de 5 à 85 % en poids du HFC-134a et de 2 à 20 % en poids du HFC-152a.

2. Compositions selon la revendication 1 **caractérisées en ce qu'**elles comprennent de 2 à 15 % en poids du HFC-152a, de 15 à 70 % en poids du 2,3,3,3 tetrafluoropropène et de 15 à 70 % en poids du HFC-134a.

3. Compositions selon la revendication 1 ou 2 **caractérisées en ce qu'**elles comprennent de 10 à 11 % en poids du HFC-152a, de 82 à 83 % en poids du 2,3,3,3 tetrafluoropropène et de 6 à 7 % en poids du HFC-134a.

4. Compositions selon l'une quelconque des revendications 1 à 3 **caractérisées en ce qu'**elles sont (quasi)azéotropiques.

5. Fluides de transfert de chaleur selon l'une quelconque des revendications précédentes .

6. Fluides de transfert de chaleur selon la revendication 5 **caractérisés en ce qu'**ils sont utilisés dans l'air conditionné et le chauffage en remplacement du R407C et HFC-134a.

7. Agents d'expansion comprenant les compositions selon l'une quelconque des revendications 1 à 4.

8. Aérosols comprenant les compositions selon l'une quelconque des revendications 1 à 4.

9. Solvants comprenant les compositions selon l'une quelconque des revendications 1 à 4.

## Patentansprüche

1. Zusammensetzungen, umfassend 10 bis 90 Gew.-% 2,3,3,3-Tetrafluorpropen, 5 bis 85 Gew.-% H-FKW 134a und 2 bis 20 Gew.-% H-FKW 152a.

2. Zusammensetzungen nach Anspruch 1, **dadurch gekennzeichnet, dass** sie 2 bis 15 Gew.-% H-FKW 152a, 15 bis 70 Gew.-% 2,3,3,3-Tetrafluorpropen und 15 bis 70 Gew.-% H-FKW 134a umfassen.

3. Zusammensetzungen nach Anspruch 1 oder 2, **dadurch gekennzeichnet, dass** sie 10 bis 11 Gew.-% H-FKW 152a, 82 bis 83 Gew.-% 2,3,3,3-Tetrafluorpropen und 6 bis 7 Gew.-% H-FKW 134a umfassen.

4. Zusammensetzungen nach einem der Ansprüche 1 bis 3, **dadurch gekennzeichnet, dass** sie (quasi)-azeotrop sind.

5. Wärmeübertragungfluide nach einem der vorhergehenden Ansprüche.

6. Wärmeübertragungfluide nach Anspruch 5, **dadurch gekennzeichnet, dass** sie beim Klimatisieren und Erwärmen als Ersatz für R407C und H-FKW 134a verwendet werden.

7. Treibmittel, umfassend die Zusammensetzungen nach einem der Ansprüche 1 bis 4.

8. Aerosole, umfassend die Zusammensetzungen nach einem der Ansprüche 1 bis 4.

9. Lösungsmittel, umfassend die Zusammensetzungen nach einem der Ansprüche 1 bis 4.

## Claims

1. Compositions comprising from 10% to 90% by weight of 2,3,3,3-tetrafluoropropene, from 5% to 85% by weight of HFC-134a and from 2% to 20% by weight of HFC-152a.

2. Compositions according to Claim 1, **characterized in that** they comprise from 2% to 15% by weight of HFC-152a, from 15% to 70% by weight of 2,3,3,3-tetrafluoropropene and from 15% to 70% by weight of HFC-134a.

3. Compositions according to Claim 1 or 2, **characterized in that** they comprise from 10% to 11% by weight of HFC-152a, from 82% to 83% by weight of 2,3,3,3-tetrafluoropropene and from 6% to 7% by weight of HFC-134a.

4. Compositions according to any one of Claims 1 to 3, **characterized in that** they are (quasi)azeotropic.

5. Heat transfer fluids according to any one of the preceding claims.

6. Heat transfer fluids according to Claim 5, **characterized in that** they are used in conditioned air and heating as a replacement for R407C and HFC-134a.

7. Blowing agents comprising the compositions according to any one of Claims 1 to 4.

8. Aerosols comprising the compositions according to any one of Claims 1 to 4.

9. Solvents comprising the compositions according to any one of Claims 1 to 4.
